# EUROPÄISCHE PATENTSCHRIFT

(11) **EP 1 376 112 B1**
(45) Veröffentlichungstag und Bekanntmachung des Hinweises auf die Patenterteilung: **21.01.2009**
(21) Anmeldenummer: 03014623.7
(22) Anmeldetag: 26.06.2003
(51) Int. Cl.: G01N 27/02

(54) **Verfahren zum Steuern eines Reinigungsvorgangs von Belägen auf einem Arbeitsteil**
Method for controlling a cleaning progress of coatings on a working member
Procédé pour le réglage des étapes de nettoyage des couches sur un outil

(30) Priorität: 27.06.2002 DE 10228811
(43) Veröffentlichungstag der Anmeldung: 02.01.2004
(73) Patentinhaber: Fraunhofer-Gesellschaft zur Förderung der angewandten Forschung e.V., 80686 München (DE)
(72) Erfinder: Endres, Hanns-Erik, 80689 München (DE); Hammerl, Eva, 83671 Benediktbeuern (DE); Drost, Stephan, 81241 München (DE); Menner, Michael, 82223 Eichenau (DE); Pfeiffer, Thomas, 80799 München (DE)
(74) Vertreter: Schoppe, Fritz

(56) Entgegenhaltungen:
- EP-A- 1 111 356
- EP-A- 1 143 240
- DD-A- 248 504
- DE-A- 10 008 481
- DE-A- 19 755 418
- US-A- 5 221 893
- US-A- 5 789 670
- US-A- 5 859 537
- US-A- 6 023 170
- US-B1- 6 239 601
- US-B1- 6 300 123
- PATENT ABSTRACTS OF JAPAN vol. 2002, no. 05, 3. Mai 2002 (2002-05-03) & JP 2002 014067 A (TOSHIBA CORP), 18. Januar 2002 (2002-01-18)
- LONERGAN ET AL: "Array-based vapor sensing using chemically, carbon balck-polymer resistors" CHEM. MATER., Bd. 8, 1996, Seiten 2298-2312, XP000979333

## Beschreibung

Die vorliegende Erfindung bezieht sich auf das Erfassen von Belägen und insbesondere auf das Erfassen einer Änderung eines Belags bei einem Reinigungsvorgang.

Bei der Verarbeitung und Herstellung von flüssigen Lebensmitteln, wie beispielsweise Milch, Bier oder Säften, kommt es in den Produktionsanlagen zur Bildung von Belägen. Das Auftreten von Belägen in Anlagen der lebensmittelverarbeitenden Industrie ist dabei insbesondere in Wärmetauschern bei Pasteurisier- und Sterilisierprozessen eine unvermeidliche Erscheinung. Es treten sowohl mineralische, organische und gemischt organisch-mineralische Beläge auf, die häufig Dicken bis zu 0,5 mm oder darüber erreichen können. Diese Beläge beeinträchtigen den Wärmeübergang der Wärmetauscher, erhöhen die Druckverluste und können sich durch steigende Keimzahlen und durch Kochgeschmack negativ auf die Produktqualität auswirken.

Die Reinigung der Anlagen zur Entfernung der Beläge von produktführenden Oberflächen erfolgt im Stand der Technik typischerweise mittels Cleaning-In-Place-Techniken (CIP-Techniken), wobei in der Regel sowohl alkalische als auch saure Reinigungschemikalien sowie Desinfektionsmittel jeweils mit einer Zwischenspülung eingesetzt werden. Die Anwendung erfolgt meist bei erhöhter Temperatur. Die CIP-Reinigung wird gemäß dem Stand der Technik in technisch festgelegten Intervallen und mit zum Teil großen Sicherheitszuschlägen bei der Reinigungsdauer durchgeführt.

Die Erfinder haben in einer Marktstudie insgesamt 368 Unternehmen aus der milchverarbeitenden Industrie und der Brauwirtschaft befragt. Nach den Ergebnissen der Befragung erfolgen in 72 % der Fälle die Reinigungsläufe in einem festen Zeitraster oder in Abhängigkeit von den Gegebenheiten des Produktionsablaufs wie Schichtwechsel, Chargenwechsel, Produktwechsel oder Produktionsende.

Es wurde eine durchschnittliche Zahl von zehn Reinigungsläufen pro Anlage und Woche und eine durchschnittliche Dauer der Reinigungsgänge von 110 Minuten ermittelt. Daraus läßt sich eine kumulierte durchschnittliche Reinigungsdauer von etwa 1000 Minuten (etwa 17 Stunden) pro Woche abschätzen. Bei einem Dreischichtbetrieb mit 5-Tage-Woche macht dies etwa 15 % der Produktionszeit aus. Diese Abschätzung stimmt gut mit der Angabe der Firmen überein, die im Durchschnitt 14 % der Anlagenbetriebszeit für die Reinigung veranschlagen.

Die Reinigung erfolgt fast ausschließlich mit CIP-Verfahren (83 %), bei denen basische, d.h. meist NaOH (1-4 %), und saure Reinigungsmittel, d.h. meist HNO₃ (1-3,5 %), in aufeinanderfolgenden Reinigungsgängen eingesetzt werden. Die Temperatur liegt im basischen Reinigungsgang zwischen 60°C und 140°C, im sauren Reinigungsgang zwischen 50°C und 80°C. Eine Überprüfung des Reinigungserfolges erfolgt nicht bei allen befragten Unternehmen (60 % Überprüfung) und wenn, dann meist durch visuelle Anlageninspektion (55 %).

Die befragten Firmen erwarten von einem Belagsensor eine bedarfsgerechte Auslösung der Reinigung und damit eine Reduzierung der Anzahl von Reinigungsgängen (65 %). Ferner wird von einem Sensor eine bessere Information über die vollständig erfolgte Reinigung erwartet und damit die Möglichkeit, unnötig lange Reinigungsgänge zu vermeiden (69 %). Beide Anforderungen halten sich etwa die Waage.

Bei der Anzahl der Reinigungen erwarten die Firmen Einsparungen von 0 %-20 % (Mittelwert 8,5 %), bei der Reinigungsdauer Einsparungen von 10 %-50 % (Mittelwert 22 %). Die erwartenden Reduzierungen der Anlagenstillstandszeiten liegen zwischen 5 % und 50 % (Mittelwert 17 %). Außerdem erwartet man Einsparungen beim Verbrauch von Reinigungsmitteln in Höhe von 10 %-30 % (Mittelwert 14 %).

Unter Bezugnahme auf die oben ausgeführte Marktstudie besteht folglich ein Bedarf nach einem Sensor, der ein Erkennen eines Reinigungsfortschritts bei einer Reinigung von Belägen in Anlagen oder Arbeitsteilen ermöglicht.

Diese Aufgabe wird durch ein Verfahren zum Steuern eines Reinigungsvorgangs nach Anspruch 1 gelöst.

Aus der Fachveröffentlichung Lonergan M., et. al.: "Array-Based Vapor Sensing Using Chemically Sensitive, Carbon Black-Polymer Resistor", Chem. Mater. 1996, 8, Seite 2298 - 2312 ist es bekannt, eine Sensorstruktur zum Erfassen von Gasen mit einer Vielzahl von unterschiedlich beschichteten kapazitiven Sensoren zu schaffen, die jeweils auf bestimmte Gase durch Veränderung ihrer Oberflächenbeschichtung ansprechen. Diese Veränderung wird bei sämtlichen kapazitiven Sensoren mit der gleichen Frequenz ausgelesen, um die Kapazitätsänderung für jeden der Sensoren zu erfassen. Aufgrund dieser bei der jeweils gleichen Frequenz ausgelesenen Kapazitätsänderungswerte wird eine Hauptkomponentenanalyse vorgenommen.

Aus der EP 1111345 A2 ist eine Positionsmesseinrichtung bekannt, die eine Inkrementalsignaldetektoranordnung umfasst, die auf zwei Inkrementalspuren anspricht, die mit unterschiedlichen Teilungsperioden versehen sind.

Die DE 197 55 418 A 1 zeigt ein Sensorelement zur Messung komplexer Impedanzen. Das Sensorelement ist mit einer Meßvorrichtung angeordnet, um beispielsweise Flüssigkeiten, Ionenkonzentrationen, einen Wassergehalt in Flüssigkeiten zu messen. Zur Messung einer komplexen Impedanz wird ein Wechselstrom eingeprägt, wobei ein Wechselspannungssignal erfaßt wird, so daß der Real- und Imaginärteil der Impedanz bestimmt werden kann.

Die US 5,789,670 offenbart einen Feuchtigkeitssensor, der interdigital angeordnete parallele leitfähige Spuren aufweist. Die leitfähigen Spuren sind an jeweiligen Enden mit einem elektrischen Anschluß verbunden. Bei Befeuchten eines auf die leitfähigen Spuren definierten Sensorfelds ändert sich der elektrische Widerstand zwischen den Sensorelementen, so daß ein Anschalten eines Scheibenwischers bewirkt wird.

Die JP 2002-014067 beschreibt eine Vorrichtung zum Erfassen einer Verschlechterung eines Schutzfilms, beispielweise einer Korrosion oder einer Verschlechterung aufgrund von Feuchtigkeit oder Ultraviolettstrahlen. An den zu erfassenden Film wird eine Spannung angelegt, so daß durch das Erfassen eines elektrischen Stroms eine Impedanz des Films bestimmt werden kann. Die Spannung wird in zwei oder mehreren unterschiedlichen Frequenzen in einem Bereich von 0,01 Hz bis 1 kHz angelegt.

Die US 6,239,601 B1 zeigt ein Bestimmen einer Dicke einer Eisschicht oder einer Mischung aus Eis und Wasser. Zum Erfassen einer Impedanz sind zwei Impedanzsensoren Vorgesehen, die bei einer ersten und zweiten Frequenz betrieben werden. Der erste Sensor weist eine erste Elektrodenanordnung auf, die Elektroden mit einem ersten Elektrodenabstand umfassen, während der zweite Sensor eine Elektrodenanordnung mit Elektroden umfaßt, die einen zweiten Elektrodenabstand umfassen. Bei zwei vorbestimmten Frequenzen wird eine Impedanz erfaßt und unter Verwendung von Meßwerten, die ohne das Vorhandensein der zu erfassenden Eisschicht bestimmt wurden, ein Verhältnis der Kapazitäten der zwei Sensoren bestimmt, welches proportional zu der Dicke der Eisschicht ist.

Die EP 1 143 240 A1 beschreibt ein Verfahren zum Bestimmen von Charakteristika einer Probenflüssigkeit, die eine Mehrzahl von Substanzen aufweist. Zum Erfassen der Charakteristika wird ein Cyklovoltagramm aufgenommen, bei dem Stromflüsse, die durch einen Potentiostaten gemessen werden, in Abhängigkeit einer angelegten Spannung aufgetragen werden. Aus einem Maximum des Stroms läßt sich eine Auskunft über die Konzentration eines Stoffes gewinnen, indem eine Hauptkomponentenanalyse der maximalen Werte durchgeführt wird.

Die US 6,300,123 B1 offenbart einen Sensor, der eine leitfähige Elektrode aufweist, auf dem eine leitende Polymerschicht erzeugt wird, um eine Bio-Affinität bezüglich bestimmter Substanzen aufzuweisen. Die Polymerschicht kann die zwei Elektroden überbrücken, zwischen denen die Impedanz gemessen wird. Zum Erfassen der zu überwachenden Substanzen wird eine Messung über einen Frequenzbereich von 0,01 Hz bis 100 kHz durchgeführt, wobei aus den erfaßten Impedanzwerten eine Bioaktivität bestimmt wird.

Die US 5,859,537 beschreibt ein Bestimmen einer Materialverschlechterung einer Überzugschicht durch Erfassen einer Impedanz derselben durch ein Elektroden-Test-System. Das Elektroden-System umfaßt eine Elektrode, die auf der Oberfläche der zu erfassenden Überzugsschicht aufgebracht wird. Die Elektrode weise eine geringere Impedanz, verglichen mit der Impedanz der Überzugsschicht, auf. Die gesamte Oberfläche der aufgebrachten Elektrode kann als eine Referenzelektrode verwendet werden, wobei eine elektrochemische Impedanz-Spektroskopie verwendet wird, um eine Korrosion zu erfassen.

Die US 5,221,893 offenbart ein Verfahren zum Diagnostizieren einer Verschlechterung eines Lackfilms, bei dem eine Impedanz des Films mittels Elektroden, die auf einer Oberfläche des Films befestigt sind, erfaßt wird. Daraufhin wird unter Verwendung von Recheneinheiten aus den gemessenen Impedanzen eine Verschlechterung des Films berechnet.

Die DE 100 08 481 A 1 beschreibt das Durchführen einer Messung zur Bestimmung einer Konsistenz von Knochenzement mittels eines Erfassens eines Imaginärteils der Dielektrizitätszahl des Knochenzements.

Die US 6,023,170 offenbart ein Verfahren zum Bestimmen eines Aushärtungsgrads eines Materials unter Verwendung einer ersten Dielektrizitätszahl, die zu einem ersten Zeitpunkt erfaßt wird, und einer zweiten Dielektrizitätszahl, die zu einem späteren Zeitpunkt erfaßt wird.

Die DD 248 504 A5 zeigt ein Verfahren zum Nachweis einer Euterentzündung, bei dem eine Milchprobe, die von einem jeweiligen Euterviertel entnommen ist, mit einem Strom beaufschlagt wird, und eine abfallende Spannung gemessen wird.

Bei einem bevorzugten Ausführungsbeispiel weist der Interdigital-Kondensator interdigital angeordnete kammförmige Elektroden auf, die einen Abstand von wenigen µm bis einige mm aufweisen können. Dies ermöglicht, dass ein an den Interdigital-Kondensator angelegtes elektrisches Feld in den auf der äußeren Oberfläche des Interdigital-Kondensators abgeschiedenen Belag eindringen kann, wodurch eine sichere Erfassung desselben ermöglicht wird. Vorzugsweise weist ein Chip, auf dem der Interdigital-Kondensator angeordnet ist, eine Fläche im Bereich von 50-500 mm² auf, wodurch ein günstiger Kompromiß einer handhabbaren Chipfläche und Dimensionen, die für eine mikroskopische Belagbildung notwendig sind, erreicht wird. Bei einem Ausführungsbeispiel weist der Chip eine Größe von 10 x 20 mm² auf.

Vorzugsweise weist der Interdigital-Kondensator eine Schicht, deren Außenoberfläche die aktive Sensoroberfläche bildet, aus einem Material auf, dessen Oberfläche sich bezüglich einer Belagsbildung vergleichbar mit den Flächen des Arbeitsteils oder der Anlage verhält, bei der eine Belagsänderung erfaßt werden soll. Bei einem bevorzugten Ausführungsbeispiel weist die Schicht SiC auf, wodurch sowohl eine repräsentative Belagsbildung als auch ein hoher chemischer und mechanischer Schutz des Interdigital-Kondensators erreicht wird.

Bei einem Ausführungsbeispiel wird eine Impedanz des Interdigital-Kondensators auf eine fortlaufende Weise, vorzugsweise alle 2 bis 10 Minuten, erfaßt. Bei der Erfassung kann vorzugsweise jeweils ein Spektrum von Impedanzen gemessen werden, beispielsweise über einen Frequenzbereich von 0,5-500 kHz. Die zeitlichen Abstände zwischen den jeweiligen Erfassungszeitpunkten wird vorzugsweise abhängig von der Änderung des Belags eingestellt, wobei bei einem typischen Meßverfahren etwa alle 2 bis 10 Minuten eine Messung der Impedanzen oder Impedanzspektren erfolgt.

Vorzugsweise wird bei einem bevorzugtem Ausführungsbeispiel zur Berechnung der Indikatorgröße eine Hauptkomponentenanalyse der Impedanzspektren verwendet, wobei für jede durchgeführte Erfassung ein Meßvektor in einem Hauptkomponentenraum, der nachfolgend auch als Hauptkomponentenvektor bezeichnet wird, berechnet wird. Unter Verwendung eines berechneten Hauptkomponentenvektors sowie einem oder mehrerer vorhergehend berechneter Hauptkomponentenvektoren, die vorhergehenden Erfassungen zugeordnet sind, kann bei diesem Ausführungsbeispiel die Indikatorgröße berechnet werden. Als Indikatorgröße kann beispielsweise ein Abstand der Hauptkomponentenvektoren oder eine Richtung eines Differenzvektors im Hauptkomponentenraum verwendet werden.

Die bei diesem Verfahren durchgeführte regelmäßige Neuberechnung der Hauptkomponententransformation erfolgt vorzugsweise ohne Rückgriff auf vorab ermittelte Meßvektoren, wodurch ein Kriterium für ein Eingreifen in den Prozessor lediglich aus der Trajektorie der Meßvektoren im Hauptkomponentenraum abgeleitet wird. Dies ermöglicht ein Steuern des Prozesses ohne daß ein a piori Wissen über den Prozess erforderlich ist. Ferner ist durch die sukzessive Durchführung der Hauptkomponentenanalyse kein kalibriertes Hauptkomponentensystem notwendig, so daß Probleme, die durch Sensordriften, unerwartete Quereinflüsse usw. entstehen können, weitgehend vermieden werden.

Bevorzugte Ausführungsbeispiele der vorliegenden Erfindung werden nachfolgend unter Bezugnahme auf die beiliegenden Zeichnungen näher erläutert. Es zeigen:
- Fig. 1: ein schematisches Blockschaltbild eines Belagsensors gemäß einem Ausführungsbeispiel der vorliegenden Erfindung;
- Fig. 2: eine schematische Darstellung eines Interdigital-Kondensatorsensors gemäß einem Ausführungsbeispiel der vorliegenden Erfindung;
- Fig. 3: eine Abbildung von Interdigital-Kondensatorsensoren gemäß einem Ausführungsbeispiel der vorliegenden Erfindung, wobei ein Interdigital-Kondensatorsensor in ein Systembauteil integriert ist;
- Fig. 4: eine Abbildung einer Versuchstrecke zur Simulation von Belagbildungen und einer Reinigung mit Wärmebad;
- Fig. 5: eine Abbildung eines Interdigital-Kondensatorsensors gemäß einem Ausführungsbeispiel der vorliegenden Erfindung mit einem angetrockneten Belag, der sich nach einem Arbeitsvorgang bildet;
- Fig. 6: eine Abbildung des Interdigital-Kondensatorsensors gemäß Fig. 5 nach einer alkalischen und sauren Reinigung;
- Fig. 7: eine Abbildung des Interdigital-Kondensatorsensors gemäß Fig. 5 bei dem eine alkalische Reinigung eines Belags durchgeführt wurde; und
- Fig. 8: ein Schaubild, bei dem der Verlauf einer Indikatorgröße in Abhängigkeit der Zeit für einen Reinigungsprozeß gezeigt ist.

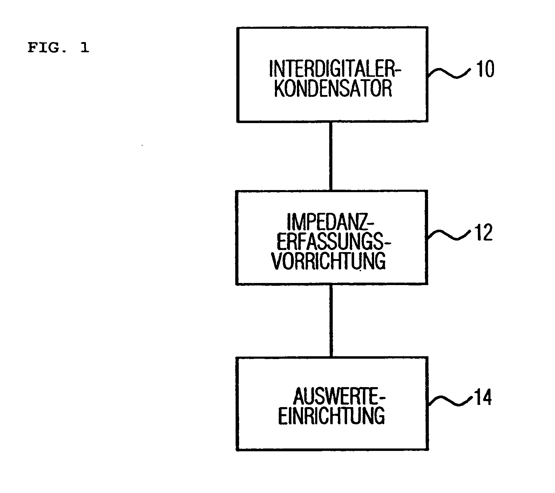

Fig. 1 zeigt ein schematisches Blockschaltbild einer Vorrichtung zum Erfassen einer Belagsänderung gemäß einem Ausführungsbeispiel der vorliegenden Erfindung. Gemäß Fig. 1 ist ein Interdigital-Kondensator 10 mit einer Impedanzerfassungsvorrichtung zum Erfassen von Impedanzwerten des Interdigital-Kondensators verschaltet. Die Impedanzerfassungsvorrichtung ist ferner mit einer Auswerteeinrichtung verbunden, die ausgebildet ist, um unter Verwendung der erfassten Impedanzwerte eine Indikatorgröße zu berechnen, die auf eine zeitliche Änderung des Belags hinweist.

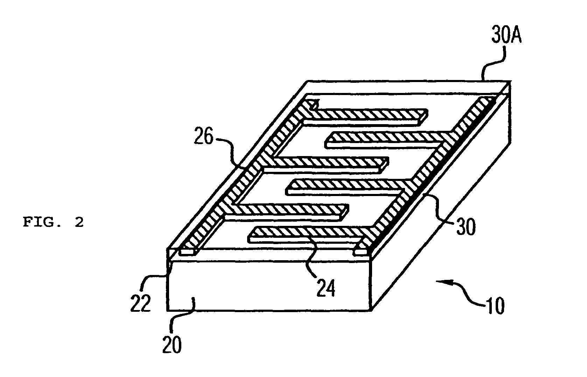

Ein Ausführungsbeispiel des Kondensators 10, der vorzugsweise ein planarer Kondensator ist, um eine Befestigung oder einen Einbau in die Wände beispielsweise von Rohrleitungen oder Behältern zu erleichtern, ist in Fig. 2 gezeigt. Der Kondensator 10 kann dabei auch direkt auf einer Leiterplatte angeordnet sein. Bei dem Ausführungsbeispiel gemäß Fig. 2 ist der Kondensator 10 als ein Interdigital-Kondensator ausgebildet, bei dem auf einem dielektrischen Substrat 20 auf einer Oberfläche 22 des dielektrischen Substrats 20 eine erste kammförmige Elektrode 24 und eine zweite kammförmige Elektrode 26 angeordnet sind. Die erste und zweite kammförmige Elektrode werden vorzugsweise in Dünnfilmtechnologie mit einer Rasterbreite von einigen 10 µm gebildet. Wie es in Fig. 2 zu erkennen ist, sind die erste und zweite kammförmige Elektrode 24 und 26 interdigital zueinander angeordnet, so daß zwischen den Elektroden eine Kapazität gebildet ist. Die Elektroden 24 und 26 sind mit einer isolierenden und chemikalienresistenten Schutzschicht 30 beschichtet, die bei diesem Ausführungsbeispiel Siliziumcarbid (SiC) aufweist. Zusätzlich kann auf dem Sensortyp ein Widerstandsmäander (nicht gezeigt) zur Temperaturmessung integriert sein.

Bei einem Betrieb wird eine äußere Oberfläche 30a der Schutzschicht 30 als sensitive Fläche verwendet, auf der ein Belag abscheidbar ist. Die Wirkungsweise des Kondensators 10 beruht darauf, daß die elektrische Impedanz Z desselben von dem dielektrischen Material des Substrats 22, der sich über dem Sensor befindlichen Flüssigkeit und dem auf der Schicht 30 abgeschiedenen Belag bestimmt wird. Ändert sich z.B. die Dicke des Belags oder dessen Zusammensetzung, so kann dies über die Messung der Impedanz Z erkannt werden. Die Eindringtiefe des elektrischen Felds in die Flüssigkeit hängt im wesentlichen von dem Elektrodenabstand ab und liegt als erste Näherung in der Größenordnung des Elektrodenabstands. Der Elektrodenabstand kann mittels bekannter Technologien im µm-Bereich variiert werden, wobei die Auswahl des Elektrodenabstands unter Berücksichtigung der Flüssigkeit und einer typischen Zusammensetzung und Dicke eines aus der Flüssigkeit abgeschiedenen Belags getroffen wird.

Bei einer Auswahl der Größe des planaren Kondensators, d.h. genauer gesagt des Chips, auf dem der Kondensator aufgebracht ist, ist zu berücksichtigen, daß sowohl eine Handhabbarkeit der Chipfläche als auch eine entsprechende Dimension für eine mikroskopische Belagbildung erforderlich sind. Bei einem Ausführungsbeispiel umfaßt die Größe des Chips 10 x 20 mm², wobei diese Flächendimension einen günstigen Kompromiß zwischen einer handhabbaren Chipfläche und den Dimensionen, die für eine mikroskopische Belagbildung notwendig sind, darstellt. Bei einem typischen Ausführungsbeispiel weist der Kondensator eine Kapazität von etwa 60 pF in Luftumgebung auf, wobei dieser Wert in wässeriger Umgebung deutlich zunimmt.

Um den Kondensator 10 als Sensor einer Vorrichtung zum Erfassen einer Belagsänderung zu verwenden kann der Kondensator 10 in dem Arbeitsteil oder der Anlage, in der eine Belagsänderung erfaßt werden soll, angeordnet oder an einer Wand befestigt werden. Typischerweise wird der Sensorchip für den Einbau auf eine Leiterplatte geklebt, kontaktiert und mit einem flüssigkeitsdichten Kunststoff so vergossen, daß nur die aktive Sensorfläche frei bleibt.

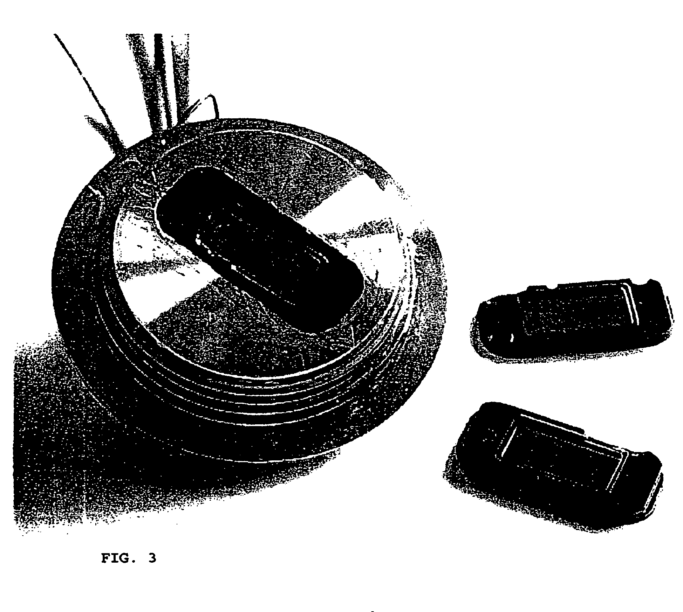

Fig. 3 zeigt eine mögliche Befestigung, wobei das fertige Sensormodul mit der aktiven Oberfläche nahezu bündig in einem bekannten Flansch, wie beispielsweise den Varivent™ - Flansch (Systembauteil der Firma GEA Tuschenhagen) eingebaut ist. Die zur Messung der Impedanz erforderlichen Kabel werden bei diesem Ausführungsbeispiel rückseitig zu einem externen Meßgerät, das beispielsweise einen bekannten Impedanzanalysator umfassen kann, herausgeführt.

Unter Bezugnahme auf Versuche, die von den Erfindern zur Untersuchung des Sensorverhaltens bei Belagsbildung und Abreinigung durchgeführt wurde, wird im folgenden ein Betrieb der Vorrichtung zur Erfassung einer Belagsänderung erklärt.

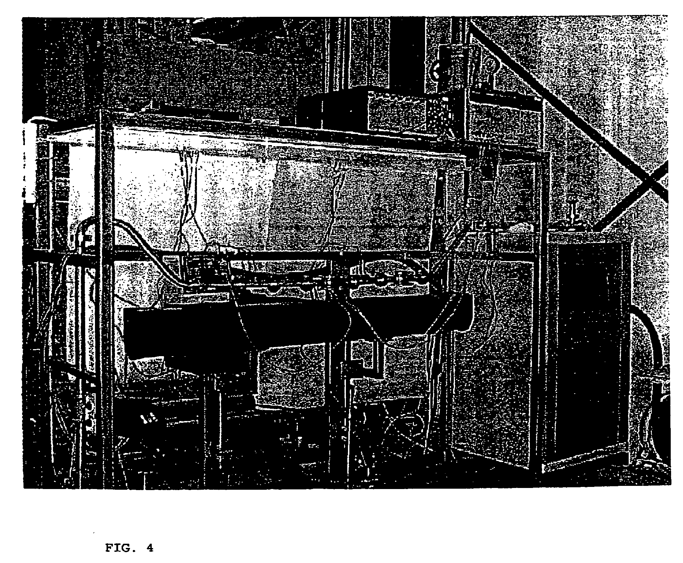

Bei den Versuchen wurde ein Versuchsstand aufgebaut, der die Verhältnisse, wie sie in einem Röhrenwärmetauscher in Bezug auf Medientemperatur, Wandtemperatur und Strömungsverhältnisse zu erwarten sind, simulieren sollte. Fig. 4 zeigt eine Abbildung des Versuchsaufbaus.

Als flüssiges Testmedium, aus dem ein Belag abgeschieden werden sollte, wurde eine Molkepulverlösung verwendet. Bei einer anschließenden Reinigung wurde ein alkalisches und ein saures Industriereinigungsmittel gewählt. Um den Verbrauch an flüssigem Versuchsmedium im Labor gering zu halten und im Interesse einer forcierten Belagsbildung wurde jedoch mit geringen Strömungsgeschwindigkeiten in der Teststrecke gearbeitet. Der Versuchsstand wurde dann dahingehend entworfen, um einen kompletten Betriebszyklus einer lebensmitteltechnischen Anlage manuell gesteuert nachzufahren. Der Betriebszyklus umfaßt dabei eine Pasteurisierung (mit Belagbildung), ein Spülen, ein alkalisches Reinigen, ein erneutes Spülen, ein saures Reinigen und ein nochmaliges Spülen.

Aufgrund der obigen Überlegungen wurde als Arbeitsteil ein DN25-Testrohr gewählt, das mit einem Mediendurchsatz von etwa 200 l/h betrieben wurde. Das DN25-Rohr stellt eine ausreichende Weite sicher, um den Sensor aufzunehmen. Ferner reicht die mittlere Strömungsgeschwindigkeit von 0,1 m/s, die sich bei einem Volumenstrom von 200 l/h einstellt, aus, um die Strömung turbulent zu halten, was für den Wärmeübergang von der Rohrwand in das Versuchsmedium und für realistische thermische Verhältnisse an der Rohrwand wichtig ist. Zur Temperierung verläuft die Versuchsstrecke in einem Wärmebad, das erheblich höhere Temperaturen annehmen kann, als das Versuchsmedium. Damit kann die charakteristische Überhitzung der Wärmetauscherrohrwand eingestellt werden.

Zur Erfassung einer Änderung eines Belags wurden während eines Versuchslaufs alle 2 bis 10 Minuten Impedanzspektren des Sensors aufgenommen. Die Messung der Impedanz Z wurde über einen Frequenzbereich von 0,5 bis 500 kHz jeweils durchgeführt. Dieser Frequenzbereich weist den Vorteil auf, daß dafür preisgünstige Schaltungen verwendet werden können. Der Fequenzbereich kann jedoch auch einen für die elektrochemische Impedanzspektroskopie üblichen Frequenzbereich, der beispielsweise 10Hz bis 1 Mhz umfaßt, umfassen. Wie es nachfolgend genauer erklärt wird, wird aus den aufgenommenen Impedanzspektren über ein Rechenverfahren der Reinigungserfolg ermittelt. Die Gesamtzahl der Spektren, die während eines Versuchs aufgenommen werden, beträgt typischerweise zwischen 50 und 100. Dies stellt sicher, daß für das Rechenverfahren eine ausreichende Datenmenge verfügbar ist. Zur Durchführung einer gleichzeitigen visuellen Kontrolle der Belagsbildung und Belagsreinigung wurde der Sensor bei einigen Versuchen in regelmäßigen Zeitabständen ausgebaut.

Obwohl eine klare und eindeutige Beziehung zwischen der Art der Beläge, ihrer Dicke und den Versuchsbedingungen nicht hergestellt werden konnte, konnte beobachtet werden, daß die Belagbildung auf der Sensorfläche keine visuell auffälligen Unterschiede zur Belagbildung auf exponierten Metallflächen, z.B. auf der Fläche des Montageflansches, aufwies. Dies zeigt, daß sich die aktive Sensoroberfläche bezüglich einer Belagsbildung und Abreinigung vergleichbar den Edelstahloberflächen des Rohrleitungssystems verhält, so daß dieselbe als repräsentative Testfläche für kritische Anlagenteile verwendet werden kann. Diese Erkenntnis macht es erst möglich, den Kondensator 10 als einen Sensor zum Erfassen einer Belagsänderung zu verwenden, um von dem Sensor und dessen meßbaren Belags- und Reinigungszustand auf den Anlagenzustand zu schließen.

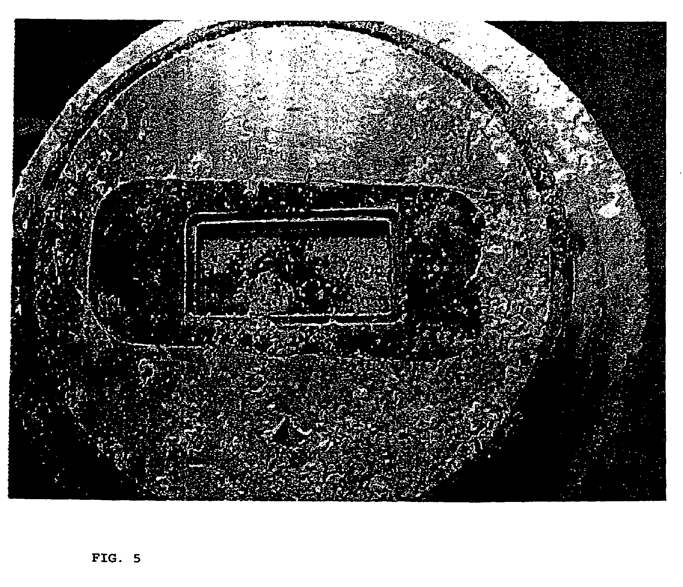

Fig. 5 zeigt einen Zustand des Sensors, nachdem derselbe eine 8 % Molkepulverlösung bei 85°C über einen Zeitraum von 120 Minuten ausgesetzt war. Wie es zu erkennen ist, bildet sich auf der Außenoberfläche des Sensors, die die aktive Oberfläche darstellt, ein Belag, der sich nicht wesentlich von einem Belag auf den Oberflächen des Flansches unterscheidet.

Bei den Reinigungsversuchen wurde in der Versuchsanlage meist eine saubere, glänzende Sensoroberfläche erreicht, an der visuell keine Verunreinigungen mehr zu erkennen waren.

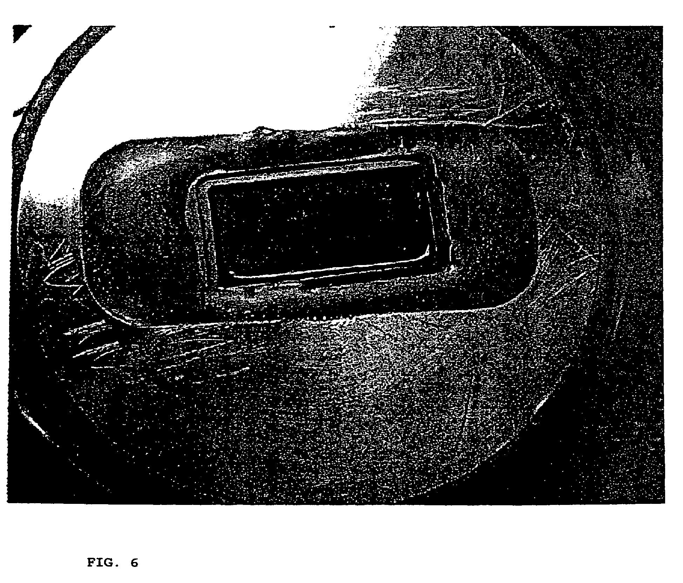

Fig. 6 zeigt eine Darstellung der Anordnung von Fig. 5, nachdem ein alkalischer und saurer Reinigungsgang durchgeführt ist. Wie es zu erkennen ist, sind sowohl die Metallflächen des Montageflansches als auch die Außenoberfläche des Sensors nach der alkalischen und sauren Reinigung visuell völlig blank.

Hinsichtlich eines Reinigungsverlaufs ist es bemerkenswert, daß der alkalische Reinigungsschritt zur Entfernung organischer Belagbestandteile sehr zeitaufwendig und kritisch ist. In Vorversuchen war es zu beobachten, daß eine Behandlungszeit von mindestens 10 Minuten bis 20 Minuten notwendig war, um eine insgesamt gute Reinigung der Oberflächen zu erzielen. Dies bestätigte sich bei den Versuchen, bei denen die Zeit für den alkalischen Reinigungssgang teilweise auf über 30 Minuten ausgedehnt werden mußte.

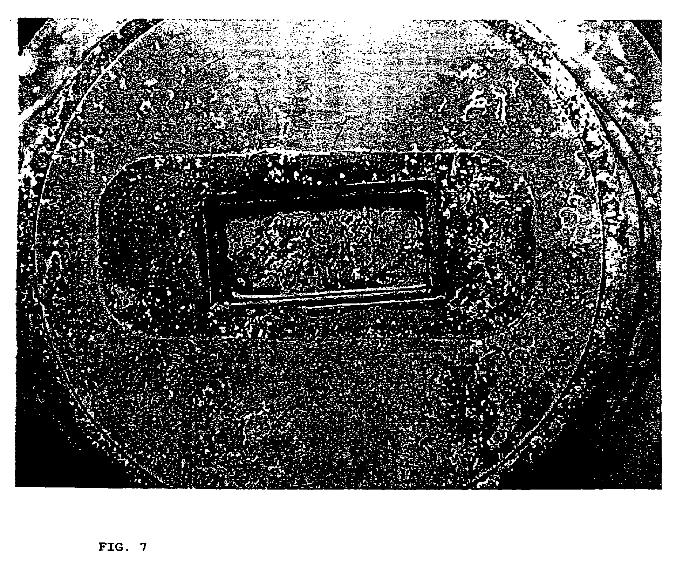

Nach Abschluß der alkalischen Reinigung sind in der Regel noch auffällige mineralische Beläge zu beobachten, wie es Fig. 7 zu entnehmen ist. Fig. 7 zeigt die Sensoranordnung des Versuchsaufbaus nach einer alkalischen Reinigung über 60 Minuten in 2 % NaOH bei 60°C. Wie zu erkennen ist, weist sowohl der metallische Flansch als auch die aktive Fläche des Sensorelements einen mineralischen Restbelag auf.

Die vorhergehend beschriebenen Versuche zeigen, daß der Kondensator 10, insbesondere aufgrund der Beschaffenheit der Schutzschicht 30, die bei diesem Ausführungsbeispiel SiC aufweist, geeignet ist, eine Belagsänderung in einem Arbeitsteil oder einer Anlage zu erfassen, da die Belagsbildung auf der Außenfläche, d.h. der aktiven Fläche des Kondensators 10 repräsentativ für die Belagsbildung auf den Metallwänden eines Arbeitsteils ist.

Im folgenden wird ein bevorzugtes Ausführungsbeispiel eines Erfassens einer Belagsänderung beschrieben. Bei diesem Ausführungsbeispiel wird durch die Impedanzerfassungsvorrichtung 12 in aufeinanderfolgenden zeitlichen Abständen ein Impedanzspektrum aufgenommen, das sich beispielsweise über einen Frequenzbereich von 0,5 bis 500 kHz erstrecken kann. Dabei wird jeweils eine Änderung zwischen den beiden letzten Messungen ausgewertet, um eine Indikatorgröße zu erhalten, die auf eine Änderung des Belags hinweist. Diese Differenzmethode ermöglicht, daß ein Endpunkt der Reinigung mit Hilfe des Sensors einfach erfaßbar ist, da das Ende der Reinigung durch einen stationären Zustand gekennzeichnet ist, bei dem an der Sensoroberfläche und den Anlagenflächen die chemischen Reinigungsvorgänge zum Stillstand gekommen sind. Bei einem Anfang der Reinigung ändern sich die von dem Sensor gelieferten Meßwerte kontinuierlich in großen Schritten, während bei einem Schluß der Reinigung die Meßwerte lediglich statistisch um einen Mittelwert schwanken. Die Differenzmethode liefert dabei ein einfaches Verfahren zur Erkennung des Endpunkts, in dem eine Bestimmung durchgeführt wird, ob eine vorbestimmte Anzahl von vorzugsweise aufeinanderfolgenden Indikatorgrößen in einem vorbestimmten Bereich liegt.

Die Berechnung der Indikatorgröße kann durch eine Hauptkomponentenanalyse erfolgen, die in einem einstufigen oder mehrstufigen Verfahren aus den Sensordaten berechnet wird. Bei diesem Verfahren werden die jeweils bei einer Erfassung gewonnenen Impedanzwerte einer Hauptkomponententransformation unterworfen, um aus den erfaßten Impedanzspektren einen Meßvektor in einem Hauptkomponentenraum zu erhalten. Weiterführende Erklärungen zur Hauptkomponentenanalyse sind beispielsweise in der EP 1 143 240 A1 zu finden. Die Hauptkomponentenberechnung wird in vorbestimmten Zeitabständen wiederholt, wobei zur Berechnung die Impedanzspektren verwendet werden, die von dem letzten Berechnungszeitpunkt bis zu dem aktuellen Berechnungszeitpunkt erfaßt wurden. Ferner kann auch eine Mittelwertbildung oder eine Auswahl der Impedanzspektren, beispielsweise indem lediglich jedes zweite Spektrum verwendet wird, durchgeführt werden. Bei der Durchführung der Hauptkomponentenanalyse können auch eine vorbestimmte Anzahl von Meßvektoren, beispielsweise die letzten n-Meßvektoren, alle zur Verfügung stehenden Meßvektoren oder gemittelte Meßvektoren verwendet werden.

Durch das wiederholte Berechnen entsteht eine Trajektorie der Meßvektoren im Hauptkomponentenraum, wobei sich das Ende des Reinigungsprozesses dadurch auszeichnet, daß die Trajektorie in einem Sättigungspunkt des Hauptkomponentenraums endet oder lediglich statistisch um denselben schwankt. Dieser Sättigungspunkt kann jedoch gewissen Einflüssen ausgesetzt sein, die das Anwenden eines Entscheidungskriterium zum Erkennen eines Reinigungsende für die Trajektorie im Hauptkomponentenraum erschweren. Beispielsweise kann sich zwischen zwei zeitlich aufeinanderliegenden Reinigungen mit gleichen Betriebsparametern eine Empfindlichkeit des Sensors aufgrund von Sensordriften vor Beginn einer Reinigung ändern, so daß sich für die beiden Reinigungen jeweils unterschiedliche Sättigungspunkte ergeben.

Um ein Verfahren zu erhalten, das stabil und weitestgehend unabhängig von solchen Einflüssen ist, wird daher eine Indikatorgröße aus den Meßvektoren im Hauptkomponentenraum berechnet, die es erlaubt, ein Ende des Reinigungsprozesses zu erkennen und eine Entscheidung bezüglich eines Eingreifens zu treffen. Dazu können verschiedene Kriterien verwendet werden, beispielsweise ein Abstandskriterium oder ein Richtungskriterium. Vorzugsweise werden dabei der aktuelle Meßvektor im Hauptkomponentenraum und der vorhergehende Meßvektor im Hauptkomponentenraum verwendet. Die Indikatorgröße wird typischerweise mit jeder neu durchgeführten Hauptkomponentenberechnung ebenfalls neu berechnet, so daß sich ein zeitlicher Verlauf von Indikatorgrößen ergibt.

Zur Entscheidung, ob ein Ende des Reinigungsprozesses vorliegt und ein Eingriff in den Reinigungsvorgang durchgeführt werden soll, wird die Indikatorgröße verwendet, beispielsweise indem eine Bestimmung durchgeführt, ob die Indikatorgrößen dauerhaft, d.h. über eine bestimmte Anzahl, in einem vorbestimmten Bereich liegen. Daraufhin kann ein Reinigungs-Ende-Signal erzeugt werden, das auf ein Ende des Reinigungsprozesses hinweist. Ferner kann, beispielsweise unter Verwendung anderer erfaßter Parameter wie beispielsweise eine Zeitdauer bis zum Erreichen des Endes oder eine erfaßte Temperatur am Endpunkt, eine Entscheidung getroffen werden, ob in den Reinigungsprozeß eingegriffen wird oder ein weiterer Prozeß durchgeführt werden soll.

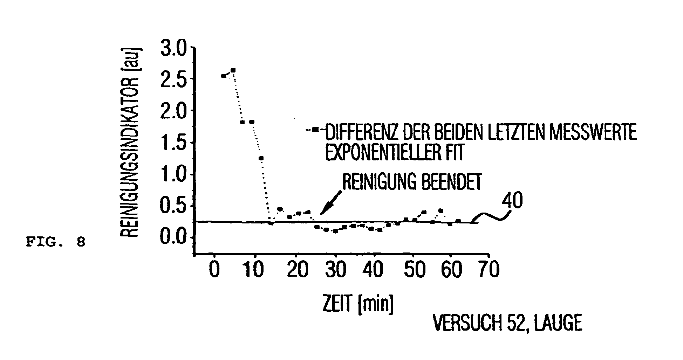

Fig. 8 zeigt eine Folge von Indikatorgrößen als Funktion der Zeit, die bei einer Laugenreinigung einer Rohrleitung ermittelt wurde. Die gewonnenen Meßwerte wurden dabei in regelmäßigen Abständen einer Hauptkomponententransformation unterworfen. Die Indikatorgröße wurde jeweils aus einer Differenz zweier aufeinanderfolgender Meßvektoren bzw. Sensordatensätzen im Hauptkomponentenraum berechnet, wobei die Mahalanobis Distanz als Abstandskriterium diente.

Wie es in Fig. 8 zu erkennen ist, zeigt sich, daß der Reinigungsfortschritt, wie bei vielen Reaktionen, einem exponentiellen Gesetz folgt. Zur Entscheidung, ob ein Eingriff in den Reinigungsvorgang durchgeführt werden soll, wird eine Bestimmung durchgeführt, ob eine Indikatorgröße dauerhaft, d.h. über eine bestimmte Anzahl, in einem vorbestimmten Bereich liegt. Beispielsweise kann man in erster Näherung einen Grenzwert vorgeben, der in Fig. 8 mit dem Bezugszeichen 40 bezeichnet ist, den das Indikatorsignal dauerhaft unterschreiten muß. Dadurch wird der Endpunkt der Reinigung angezeigt, der bei dem Schaubild gemäß Fig. 8 nach etwa 25 Minuten erreicht ist. Dieser Zeitpunkt deckt sich mit dem visuell festgestellten Endpunkt der Laugenreinigung.

Ein wesentlicher Vorteil der oben erklärten automatischen Erkennung des Reinigungsendes besteht in der Verwendung der Differenzmethode, wodurch eine Kalibrierung nicht nötig ist. Das System ist selbstkalibrierend und kann Störeinflüsse, die beispielsweise durch unterschiedliche Reinigungsmittel oder Variationen der flüssigen Lebensmittel auftreten können, in gewissem Rahmen ausgleichen.

Dank moderner numerischer Verfahren können die für die Auswertung notwendigen Berechnungen auch auf kleineren Mikrocontrollern durchgeführt werden, da der ausführbare Programmcode unter MS-DOS gerade eine Größe von 54 kB aufweist. Bei Reinigungsprozessen im Bereich von 20-60 Minuten sind Taktzeiten von einer Minute für eine Meßwertaufnahme und Berechnung völlig ausreichend. Für die Meßwertaufnahme stehen auch einfachere Schaltungen zur Verfügung, da weder die Genauigkeit noch der Leistungsumfang von Labormeßgeräten für die Prozeßmeßtechnik notwendig ist.

## Patentansprüche

1. Verfahren zum Steuern eines Reinigungsvorgangs von Belägen auf einem Arbeitsteil mit einem Interdigital-Kondensator (10) mit einer Außenfläche (30a), auf der ein Belag abscheidbar ist, so daß eine Impedanz des Interdigital-Kondensators (10) von dem abgeschiedenen Belag abhängt; einer Impedanzerfassungsvorrichtung (12) zum Erfassen von Impedanzwerten des Interdigital-Kondensators (10); und einer Auswerteeinrichtung (14) zum Berechnen einer Indikatorgröße, die auf eine zeitliche Änderung des Belags hinweist, unter Verwendung der erfaßten Impedanzwerte; wobei das Verfahren folgende Schritte aufweist:
wiederholtes Erfassen von Impedanzwerten des Interdigital-Kondensators (10) während des Reinigungsvorgangs;
Berechnen einer Indikatorgröße unter Verwendung der erfaßten Impedanzwerte mittels einer Hauptkomponentenanalyse;
Erkennen eines Endes des Reinigungsvorgang unter Verwendung der Indikatorgröße.

2. Verfahren nach Anspruch 1, bei der der Interdigital-Kondensator eine Schicht (30) aufweist, die SiC aufweist, wobei eine Oberfläche (30a) der Schicht die äußere Fläche bildet.

3. Verfahren nach Anspruch 1 oder 2, bei der die Impedanzwerte ein Impedanzspektrum umfassen.

4. Verfahren nach Anspruch 3, bei dem das Impedanzspektrum in einem Frequenzbereich von 0,5-500 kHz erfaßt wird.

5. Verfahren nach einem der Ansprüche 1-4, bei dem die Indikatorgröße unter Verwendung der beiden zuletzt erfaßten Impedanzwerte berechnet wird.

6. Verfahren nach Anspruch 1, bei dem die Indikatorgröße mittels eines Vergleichs zweier Meßvektoren in einem Hauptkomponentenraum bestimmt wird.

7. Verfahren nach Anspruch 6, bei dem die Indikatorgröße mittels einer Hauptkomponentenanalyse berechnet wird, bei der alle erfaßten Impedanzwerte, eine Auswahl der erfaßten Impedanzwerte oder gemittelte Impedanzwerte verwendet werden.

8. Verfahren nach Anspruch 6 oder 7, bei demdie Indikatorgröße mittels eines Vergleichens zweier Meßvektoren in einem Hauptkomponentenraum berechnet wird.

9. Verfahren nach. Anspruch 8, bei dem die Indikatorgröße mittels eines Abstands zweier Meßvektoren in dem Hauptkomponentenraum und/oder einer Richtung eines Differenzvektors zweier Meßvektoren in dem Hauptkomponentenraum berechnet wird.

## Claims

1. Method of controlling a cleaning process of coatings on a working member with an interdigital capacitor (10) with an outer face (30a) on which a coating can be deposited so that an impedance of the interdigital capacitor (10) depends on the deposited coating; an impedance sensing device (12) for sensing impedance values of the interdigital capacitor (10); and evaluation means (14) for calculating an indicator quantity indicating a temporal change of the coating, using the sensed impedance values; the method comprising:
repeatedly sensing impedance values of the interdigital capacitor (10) during the cleaning process;
calculating an indicator quantity using the sensed impedance values by means of a principal component analysis;
recognizing an end of the cleaning process using the indicator quantity.

2. Method according to claim 1, wherein the interdigital capacitor comprises a layer (30) comprising SiC, wherein a surface (30a) of the layer forms the outer face.

3. Method according to claim 1 or 2, wherein the impedance values include an impedance spectrum.

4. Method according to claim 3, wherein the impedance spectrum is sensed in a frequency range of 0.5-500 kHz.

5. Method according to one of claims 1-4, wherein the indicator quantity is calculated using the two last sensed impedance values.

6. Method according to claim 1, wherein the indicator quantity is determined by means of a comparison of two measurement vectors in a principal component space.

7. Method according to claim 6, wherein the indicator quantity is calculated by means of a principal component analysis in which all sensed impedance values, a selection of the sensed impedance values, or averaged impedance values are used.

8. Method according to claim 6 or 7, wherein the indicator quantity is calculated by means of comparing two measurement vectors in a principal component space.

9. Method according to claim 8, wherein the indicator quantity is calculated by means of a distance of two measurement vectors in the principal component space and/or a direction of a difference vector of two measurement vectors in the principal component space.

## Revendications

1. Procédé pour le réglage d'une opération de nettoyage de couches sur un outil avec un condensateur interdigital (10) avec une face extérieure (30a) sur laquelle peut se déposer une couche, de sorte qu'une impédance du condensateur interdigital (10) dépend de la couche déposée; un dispositif de détection d'impédance (12) destiné à détecter les valeurs d'impédance du condensateur interdigital (10); et un moyen d'évaluation (14) destiné à calculer une grandeur d'indicateur, qui indique une variation dans le temps de la couche, à l'aide des valeurs d'impédance détectées; le procédé présentant les étapes suivantes consistant à:
détecter de manière répétée les valeurs d'impédance du condensateur interdigital (10) pendant l'opération de nettoyage;
calculer une grandeur d'indicateur à l'aide des valeurs d'impédance détectées au moyen d'une analyse de composants principaux;
reconnaître une fin de l'opération de nettoyage à l'aide de la grandeur d'indicateur.

2. Procédé selon la revendication 1, dans lequel le condensateur interdigital présente une couche (30) présentant du SiC, une surface (30a) de la couche constituant la face extérieure.

3. Procédé selon la revendication 1 ou 2, dans lequel les valeurs d'impédance comprennent un spectre d'impédance.

4. Procédé selon la revendication 3, dans lequel le spectre d'impédance est détecté dans une plage de fréquences de 0,5 à 500 kHz.

5. Procédé selon l'une des revendications 1 à 4, dans lequel la grandeur d'indicateur est calculée à l'aide des deux valeurs d'impédance détectées en dernier lieu.

6. Procédé selon la revendication 1, dans lequel la grandeur d'indicateur est déterminée au moyen d'une comparaison entre deux vecteurs de mesure dans un espace de composants principaux.

7. Procédé selon la revendication 6, dans lequel la grandeur d'indicateur est calculée au moyen d'une analyse de composants principaux dans laquelle sont utilisées toutes les valeurs d'impédance détectées, une sélection des valeurs d'impédance détectées ou les valeurs d'impédance ramenées à une moyenne.

8. Procédé selon la revendication 6 ou 7, dans lequel la grandeur d'indicateur est calculée au moyen d'une comparaison entre deux vecteurs de mesure dans un espace de composants principaux.

9. Procédé selon la revendication 8, dans lequel la grandeur d'indicateur est calculée au moyen d'une distance entre deux vecteurs de mesure dans l'espace de composants principaux et/ou d'une direction d'un vecteur de différence entre deux vecteurs de mesure dans l'espace de composants principaux.
